# EUROPÄISCHE PATENTSCHRIFT

(11) **EP 0 723 810 B1**
(45) Veröffentlichungstag und Bekanntmachung des Hinweises auf die Patenterteilung: **07.11.2001**
(21) Anmeldenummer: 96100677.2
(22) Anmeldetag: 18.01.1996
(51) Int. Cl.: B01J 23/52, B01J 23/58, C07C 67/055

(54) **Katalysator, Verfahren zu seiner Herstellung und seine Verwendung für die Produktion von Vinylacetatmonomer**
Catalyst, his preparation process and use thereof to produce vinyl acetate monomer
Catalyseur, procédé pour sa préparation et son utilisation pour la production du monomère d'acétate de vinyle

(30) Priorität: 23.01.1995 DE 19501891
(43) Veröffentlichungstag der Anmeldung: 31.07.1996
(60) Teilanmeldung: 01101510.4
(73) Patentinhaber: Degussa AG, 40474 Düsseldorf (DE)
(72) Erfinder: Tacke, Thomas Dr., D-61381 Friedrichsdorf (DE); Müller, Herbert, Dr., D-63755 Alzenau (DE); Ohlrogge, Uwe, Dr., D-63456 Hanau (DE); Daly, Francis P., Dr., Morray, KY 41701 (US); Lansink-Rotgerink, Hans, Dr., D-63864 Glattbach (DE); Krause, Helmfried, D-63517 Rodenbach (DE)

(56) Entgegenhaltungen:
- EP-A- 0 431 478
- DE-A- 2 339 831
- GB-A- 1 434 738
- JP-A- 52 145 416
- JP-A- 59 177 136
- US-A- 3 939 199
- US-A- 4 087 622
- US-A- 4 321 409

## Beschreibung

Die vorliegende Erfindung betrifft einen Trägerkatalysator für die Produktion von Vinylacetatmonomer (VAM), welcher auf einem Träger aus Siliziumdioxid, Alumosilikat oder Aluminiumoxid als katalytisch aktive Komponenten Palladium, Gold und Alkaliverbindungen enthält, sowie ein Verfahren zu seiner Herstellung und seine Verwendung.

Gold, Palladium und Alkaliverbindungen enthaltende Trägerkatalysatoren werden für die Produktion von Vinylacetat verwendet. Dazu werden Ethen, Essigsäure und molekularer Sauerstoff bzw. Luft in der Gasphase, gegebenenfalls unter Zusatz von Inertgasen, bei Temperaturen zwischen 100 und 250°C und gewöhnlichem oder erhöhtem Druck in Gegenwart des Trägerkatalysators zur Reaktion gebracht.

Ein solches Produktionsverfahren wird in den Patentschriften DE 16 68 088 und US 4,048,096 beschrieben. Diese Patentschriften offenbaren auch ein Verfahren zur Herstellung der Gold, Palladium und Alkaliverbindungen enthaltenden Trägerkatalysatoren. Je nach Ausführungsform werden Katalysatoren mit homogener Edelmetallverteilung über den Trägerquerschnitt und mit mehr oder weniger ausgeprägtem Schalenprofil erhalten.

Diese Katalysatoren werden gewöhnlich durch Imprägnieren der Träger mit einer basischen Lösung und einer Gold- und Palladiumsalze enthaltenden Lösung erhalten, wobei die Imprägnierungen gleichzeitig oder nacheinander, mit oder ohne Zwischentrocknung erfolgen. Anschließend wird der Träger zur Entfernung gegebenenfalls vorhandener Chloridanteile gewaschen. Vor oder nach dem Waschen werden die auf dem Träger ausgefällten unlöslichen Edelmetallverbindungen reduziert. Die so erhaltene Katalysatorvorstufe wird getrocknet und zur Aktivierung des Katalysators mit Alkaliacetaten oder Alkaliverbindungen imprägniert, die sich unter den Reaktionsbedingungen bei der Produktion von Vinylacetatmonomer ganz oder teilweise in Alkaliacetate umwandeln. Bevorzugte Alkaliverbindungen sind Kaliumverbindungen, insbesondere Kaliumacetat.

Die Reduktion des Katalysators kann in der wäßrigen Phase oder in der Gasphase vorgenommen werden. Zur Reduktion in der wäßrigen Phase eignen sich zum Beispiel Formaldehyd oder Hydrazin. Die Reduktion in der Gasphase kann mit Wasserstoff beziehungsweise Formiergas (95 Vol.-% N₂ + 5 Vol.-% H₂) oder Ethen vorgenommen werden. Gemäß der EP 0 634 209 erfolgt die Reduktion mit Wasserstoff bei Temperaturen zwischen 40 und 260°C, bevorzugt zwischen 70 und 200°C. Häufig wird der Katalysator jedoch erst nach der Aktivierung mit Alkaliacetat direkt im Produktionsreaktor mit Ethen reduziert.

Im Produktionsprozeß wird der Katalysator erst langsam mit den Reaktanden belastet. Während dieser Anfahrphase erhöht sich die Aktivität des Katalysators und erreicht gewöhnlich erst nach Tagen oder Wochen ihr endgültiges Niveau.

Das Dokument EP-A 0431478 beschreibt ein Verfahren zur Herstellung von Vinylacetat in der Gasphase aus Ethylen, Essigsäure und Sauerstoff an einem Katalysator, der Palladium und Gold auf einem Träger enthält. Der Träger besteht aus SiO₂ oder SiO₂ / Al₂O₃-Gemisch, wobei die Trägerteilchen mit Hilfe von Li-, Mg-, Al-, Zn- oder Mn- Salzen einer C₂-C₂₀-Carboxylsäure als Bindemittel gepreßt werden.

Aufgabe der vorliegenden Erfindung ist es, einen Trägerkatalysator für die Produktion von Vinylacetatmonomer anzugeben, welcher bei gleicher beziehungsweise verbesserter Selektivität eine höhere Aktivität als konventionelle Katalysatoren aufweist. Weiterhin soll ein Verfahren zur Herstellung des Katalysators angegeben werden.

Diese Aufgabe wird durch einen Trägerkatalysator gelöst, welcher auf einem Träger aus Siliziumdioxid, Alumosilikat oder Aluminiumoxid als katalytisch aktive Komponenten Palladium, Gold und Alkaliacetat enthält. Der Katalysator mindestens ein Element aus der Gruppe Zirkon und/oder Titan enthält.

Der Katalysator kann nach den aus dem Stand der Technik bekannten Verfahren hergestellt werden. Die zusätzlichen Elemente werden dabei durch eine Vorbehandlung des Trägers in den Träger eingebracht. Diese Vorbehandlung, im folgenden auch als Modifizierung bezeichnet, besteht aus einer Imprägnierung des Trägers mit Salzen, welche als Kationen Elemente der Gruppe Zirkon und/oder Titan und als Anionen Elemente der Gruppe VIIA oder komplexe Anionen wie Nitrat, Sulfat, Carbonat oder Anionen organischer Säuren wie Acetat und Lactat enthalten. Zur Imprägnierung können auch Organometallverbindungen, insbesondere Alkoholate, oder andere Vorstufen dieser Elemente verwendet werden. Anschließend wird der vorbehandelte Träger getrocknet und bei Temperaturen von wenigstens 160°C calciniert.

Es hat sich als günstig erwiesen, die Träger durch die Vorbehandlung mit den Kationen in einer Menge von 0,1 bis 25 Gew.-% bezogen auf das Gewicht des Trägers vorzubelegen. Es werden Kationen aus der Gruppe Zirkon und/oder Titan in Form ihrer wasserlöslichen Salze (Acetate, Nitrate, Chloride) verwendet. Die Träger können mit einzelnen Kationen oder mit beliebigen Kombinationen von Kationen aus den genannten Gruppen des Periodensystems vorbehandelt werden.

Als Trägermaterial für den Katalysator eignen sich Siliziumdioxid, Alumosilikate oder Aluminiumoxid. Bei Siliziumdioxid kann es sich um gefällte oder auch durch Flammenhydrolyse von Siliziumtetrachlorid erhaltene sogenannte pyrogene Kieselsäure handeln. Wichtig ist, daß die Katalysatorträger unter den Reaktionsbedingungen des katalytischen Prozesses, insbesondere unter dem Einfluß von Essigsäure, ihre mechanische Festigkeit behalten. Vorteilhaft sind solche Trägermaterialien, die eine spezifische Oberfläche größer als 10 m²/g aufweisen, insbesondere eine spezifische Oberfläche von 50 - 250 m²/g.

Die Katalysatorträger können als Strangpreßlinge, Tabletten, Ringe oder in anderen für Festbettkatalysatoren üblichen Formen vorliegen. Die äußeren Abmessungen liegen gewöhnlich im Bereich zwischen 2 und 15 mm. Im Falle von pyrogener Kieselsäure eignen sich zum Beispiel die in der DE 38 03 895 C1 und DE 39 12 504 A1 beschriebenen Preßlinge.

Nach Trocknen und Calcinieren der vorbehandelten Katalysatorträger werden sie mit einer Palladium und Gold enthaltenden Lösung imprägniert. Gleichzeitig mit der edelmetallhaltigen Lösung oder in beliebiger Reihenfolge nacheinander werden die Katalysatorträger mit einer basischen Lösung imprägniert, welche ein oder mehrere basische Verbindungen enthalten kann. Die basische Verbindung oder Verbindungen dienen zur Überführung des Palladiums und Golds in ihre Hydroxide.

Die Verbindungen in der basischen Lösung können aus Alkalihydroxiden, Alkalibicarbonaten, Alkalicarbonaten, Alkalisilikaten oder Mischungen davon bestehen. Bevorzugt werden Kaliumhydroxid und Natriumhydroxid verwendet.

Zur Herstellung der edelmetallhaltigen Lösung können als Palladiumsalze beispielsweise Palladiumchlorid, Natrium- bzw. Kaliumpalladiumchlorid oder Palladiumnitrat verwendet werden. Als Goldsalze eignen sich Gold(III)-chlorid und Tetrachlorogold(III)-säure. Vorzugsweise wird von Kaliumpalladiumchlorid beziehungsweise Natriumpalladiumchlorid und Tetrachlorogoldsäure ausgegangen.

Das Imprägnieren des Trägers mit der basischen Lösung beeinflußt die Abscheidung der Edelmetalle im Trägermaterial. Die basische Lösung kann entweder gleichzeitig mit der Edelmetallösung oder in beliebiger Reihenfolge mit dieser Lösung mit dem Katalysatorträger in Kontakt gebracht werden. Bei aufeinanderfolgender Imprägnierung des Trägers mit den beiden Lösungen kann nach dem ersten Imprägnierschritt eine Zwischentrocknung vorgenommen werden.

Bevorzugt werden die Katalysatorträger zuerst mit der basischen Verbindung imprägniert. Die anschließende Imprägnierung mit der Palladium und Gold enthaltenden Lösung führt zur Ausfällung von Palladium und Gold in einer oberflächlichen Schale auf den Katalysatorträgern. Die umgekehrte Reihenfolge der Imprägnierungen führt im allgemeinen zu einer mehr oder weniger homogenen Verteilung der Edelmetalle über den Querschnitt der Katalysatorträger. Bei geeigneter Verfahrensführung können jedoch auch bei umgekehrter Imprägnier-Reihenfolge Katalysatoren mit definierter Schale erhalten werden (siehe z. B. US 4,048,096). Katalysatoren mit homogener oder nahezu homogener Edelmetall-Verteilung weisen im allgemeinen eine geringere Aktivität und Selektivität auf.

Katalysatoren mit Schalendicken von unter 1 mm, bevorzugt von etwa unter 0,5 mm, sind besonders geeignet. Die Schalendicke wird durch die Menge der auf das Trägermaterial aufgebrachten basischen Verbindung relativ zur gewünschten Menge der Edelmetalle beeinflußt. Je höher dieses Verhältnis ist, desto geringer wird die Dicke der sich ausbildenden Schale. Das für eine gewünschte Schalendicke erforderliche Mengenverhältnis von basischer Verbindung zu den Edelmetallverbindungen hängt von der Beschaffenheit des Trägermaterials sowie von der gewählten basischen Verbindung und den Edelmetallverbindungen ab. Das erforderliche Mengenverhältnis wird zweckmäßigerweise durch wenige Vorversuche ermittelt. Die sich ergebende Schalendicke kann dabei in einfacher Weise durch Aufschneiden der Katalysatorteilchen ermittelt werden.

Die minimal notwendige Menge der basischen Verbindung ergibt sich aus der stöchiometrisch berechneten Menge an Hydroxidionen, die zur Überführung des Palladiums und Golds in die Hydroxide benötigt werden. Als Richtwert gilt, daß die basische Verbindung für eine Schalendicke von 0,5 mm in einem 1 bis 10-fachen stöchiometrischen Überschuß angewendet werden muß.

Die Katalysatorträger werden nach dem Verfahren der Porenvolumenimprägnierung mit den basischen Verbindungen und den Edelmetallsalzen belegt. Wird mit Zwischentrocknung gearbeitet, wählt man die Volumina der beiden Lösungen so, daß sie jeweils etwa 90 bis 100 % der Aufnahmekapazität des Trägermaterials entsprechen. Wird auf die Zwischentrocknung verzichtet, so muß die Summe der Einzelvolumina der beiden Imprägnierlösungen der obigen Bedingung entsprechen, wobei die Einzelvolumina im Verhältnis von 1 : 9 bis 9 : 1 zueinander stehen können. Bevorzugt wird ein Volumenverhältnis von 3 : 7 bis 7 : 3, insbesondere von 1 : 1, angewendet. Als Lösungsmittel wird in beiden Fällen bevorzugt Wasser verwendet. Es können aber auch geeignete organische oder wäßrig-organische Lösungsmittel eingesetzt werden.

Die Umsetzung der Edelmetallsalzlösung mit der basischen Lösung zu unlöslichen Edelmetallverbindungen erfolgt langsam und ist je nach Präparationsmethode im allgemeinen erst nach 1 bis 24 Stunden abgeschlossen. Danach werden die wasserunlöslichen Edelmetallverbindungen mit Reduktionsmitteln behandelt. Es kann eine Naßreduktion zum Beispiel mit wäßrigem Hydrazinhydrat oder eine Gasphasenreduktion mit Wasserstoff, Ethen oder auch Methanoldämpfen vorgenommen werden. Die Reduktion kann bei Normaltemperatur oder erhöhter Temperatur und bei Normaldruck oder erhöhtem Druck erfolgen. Bevorzugt wird eine Naßreduktion mit wäßrigem Hydrazinhydrat oder eine Gasphasenreduktion mit Formiergas angewendet.

Vor beziehungsweise nach der Reduktion der Edelmetallverbindungen wird das auf dem Träger gegebenenfalls vorhandene Chlorid durch eine gründliche Waschung entfernt. Nach der Waschung sollte der Katalysator weniger als 500, besser weniger als 200 ppm, Chlorid enthalten.

Die nach der Reduktion erhaltene Katalysatorvorstufe wird getrocknet und abschließend mit Alkaliacetaten oder Alkaliverbindungen imprägniert, die sich unter den Reaktionsbedingungen bei der Produktion von Vinylacetatmonomer ganz oder teilweise in Alkaliacetate umwandeln. Vorzugsweise wird mit Kaliumacetat imprägniert. Hierbei wird wieder bevorzugt die Porenvolumenimprägnierung angewendet, das heißt die benötigte Menge Kaliumacetat wird in einem Lösungsmittel, vorzugsweise Wasser, gelöst, dessen Volumen etwa der Aufnahmekapazität des vorgelegten Trägermaterials für das gewählte Lösungsmittel entspricht. Dieses Volumen ist etwa gleich dem Gesamtporenvolumen des Trägermaterials.

Der fertige Katalysator wird anschließend bis auf eine Restfeuchte von weniger als 2 % getrocknet. Die Trocknung kann an Luft, gegebenenfalls auch unter Stickstoff als Inertgas, erfolgen.

Für die Synthese von Vinylacetatmonomer ist es zweckmäßig, den Katalysator mit 0,3 bis 4, vorzugsweise 0,5 bis 3 Gew.-% Palladium, 0,1 bis 2, vorzugsweise 0,2 bis 1,5 Gew.-% Gold und 1 bis 10, vorzugsweise 3,5 bis 10 Gew.-% Kaliumacetat, jeweils bezogen auf das Gewicht des eingesetzten Trägers, zu belegen. Im Fall von Katalysatorträgern mit einer Schüttdichte von 500 g/l entsprechen diese Konzentrationsangaben volumenbezogenen Konzentrationen von 1,5 bis 20 g/l Palladium, 0,5 bis 10 g/l Gold und 5 bis 50 g/l Kaliumacetat. Zur Anfertigung der Imprägnierlösungen werden die entsprechenden Mengen der Palladium- und Goldverbindungen in einem Volumen Wasser gelöst, welches etwa 90 bis 100 % der Wasseraufnahmekapazität des vorgelegten Trägermaterials entspricht. Ebenso wird bei der Anfertigung der basischen Lösung verfahren.

Zur erfindungsgemäßen Vorbehandlung der Katalysatorträger mit den genannten Kationen wird ebenfalls die Porenvolumenimprägnierung eingesetzt. Danach werden die Katalysatorträger bei erhöhten Temperaturen getrocknet und bei Temperaturen von 160 bis 800, bevorzugt 170 bis 700°C, calciniert.

Die auf vorbehandelten Trägern aufgebrachten Katalysatoren weisen eine höhere Aktivität bei der Herstellung von Vinylacetat als konventionelle Katalysatoren auf. Darüber hinaus wird bei den vorbehandelten Katalysatoren eine verbesserte Edelmetallhaftung beobachtet. Bei Verwendung von nicht vorbehandelten Katalysatorträgern treten beim Auswaschen der Chloride nach der Reduktion der Edelmetallverbindungen Edelmetallverluste auf. Diese Verluste betragen für Gold typischerweise etwa 10 % und für Palladium etwa 6 %. Demgegenüber verringern sich die Goldverluste auf ca. 6 % und die Palladiumverluste auf nur ca. 3 %, wenn die Katalysatorträger erfindungsgemäß vorbehandelt werden.

Überraschenderweise wird bei den erfindungsgemäßen Katalysatoren auch eine erhöhte Einzelkornfestigkeit beobachtet. Sie liegt bei den erfindungsgemäßen Trägerkatalysatoren bei 70 N (Beispiel 2) beziehungsweise 78 N (Beispiel 1), während die konventionell hergestellten Trägerkatalysatoren nur eine Einzelkornfestigkeit von etwa 48 N aufweisen (Vergleichsbeispiel 1).

Auch die Edelmetalldispersion ist bei den erfindungsgemäßen Katalysatoren verbessert. So wird zum Beispiel bei den erfindungsgemäßen Katalysatoren ein CO-Wert (CO-Adsorption durch CO-Puls-Chemiesorption) als Maß für die Edelmetalldispersion von 0,186 ml CO/g Katalysator (Beispiel 1) gemessen, während der CO-Wert bei konventionellen Katalysatoren nur bei 0,158 ml CO/g Katalysator (Vergleichsbeispiel 1) liegt. Die Ursache für die verbesserte Dispersion der katalytisch aktiven Edelmetalle könnte die Beobachtung sein, daß die spezifische Oberfläche der erfindungsgemäß vorbehandelten Katalysatorträger sich durch die Imprägnierung mit der basischen Lösung, insbesondere mit Natronlauge oder Kalilauge, nicht verringert. Dagegen zeigen nicht vorbehandelte Träger bei Einwirkung der basischen Lösung eine zum Teil drastische Verminderung der spezifischen Oberfläche. Damit verbunden ist eine entsprechende Erniedrigung der Edelmetalldispersion der Katalysatoren.

Eine weitere vorteilhafte Ausgestaltung der Erfindung erhält man, wenn der Katalysator in der Gasphase mit einem wasserstoffhaltigen Gas bei erhöhten Temperaturen reduziert wird. Die Gasphasenreduktion von Katalysatoren für die Synthese von Vinylacetatmonomer ist zwar schon bekannt. Sie wird jedoch bei relativ niedrigen Temperaturen vorgenommen. So schreibt zum Beispiel die US 4,370,492 eine Reduktion bei Temperaturen zwischen 40 und 260, bevorzugt zwischen 70 und 200°C, vor.

Überraschenderweise zeigte sich, daß Katalysatoren, die im Temperaturbereich zwischen 300 und 550, vorzugsweise zwischen 350 und 500°C mit Formiergas reduziert werden, deutlich höhere Aktivitäten für die Synthese von Vinylacetatmonomer bei gleichzeitig erhöhter Selektivität aufweisen. Dies gilt insbesondere auch für Katalysatoren, deren Träger nicht mit Kationen vorbehandelt wurden.

### Vergleichsbeispiel 1

Es wurde ein konventioneller Palladium-Gold-Kaliumacetat-Katalysator auf dem Alumosilikatträger KA 160 von Südchemie hergestellt. Die Trägerteilchen sind kugelförmig mit einem Durchmesser von etwa 5 mm und besitzen eine spezifische Oberfläche von 160 bis 175 m²/g, eine Schüttdichte von 600 g/l und ein Gesamtporenvolumen von 0,68 cm³/g. Die Konzentrationen der Imprägnierlösungen wurden so gewählt, daß der fertige Katalysator 3,3 g Palladium, 1,5 g Gold und 30 g Kaliumacetat pro Liter Schüttvolumen des Katalysatorträgers enthielt, was einer Konzentration von 0,55 Gew.-% Palladium, 0,25 Gew.-% Gold und 5 Gew.-% Kaliumacetat, bezogen auf das Gewicht des verwendeten Trägers, entsprach.

In einem ersten Schritt wurden die Träger zunächst mit einer Lösung von Kaliumhydroxid imprägniert. Die Konzentration der Lösung an Kaliumhydroxid war so bemessen, daß nach dem Imprägnieren ein stöchiometrischer Überschuß an Kaliumhydroxid auf dem Träger von 620 % vorlag.

Nach dem Trocknen der Katalysatorträger wurden sie mit einer wäßrigen Lösung aus Tetrachlorogoldsäure und Kaliumpalladiumchlorid imprägniert. Nach 20 Stunden wurden die unlöslichen Edelmetallverbindungen in der wäßrigen Phase mit Hydrazinhydrat während der Dauer von 4 Stunden reduziert. Danach wurden die Katalysatorträger chloridfrei gewaschen und getrocknet, bevor sie mit einer Kaliumacetat-lösung imprägniert und erneut getrocknet wurden. Vor der Imprägnierung mit Kaliumacetat betrug die spezifische Oberfläche des Katalysators nach DIN 66 132 nur noch 60 -70 m²/g. Durch die Imprägnierung beziehungsweise Aktivierung mit Kaliumacetat ging die spezifische Oberfläche des Katalysators auf 41 m²/g zurück.

Die CO-Adsorption des Katalysators vor der Aktivierung lag bei 0,158 ml CO/g Katalysator. Die Bruchfestigkeit bzw. Einzelkornfestigkeit oder Druckfestigkeit des aktivierten Katalysators wurde zu 48 N bestimmt (bei radialer Messung). Die Dicke seiner edelmetallhaltigen äußeren Schale betrug 0,3 mm.

### Referenz - Beispiel 1

Es wurde ein Palladium-Gold-Kaliumacetat-Katalysator wie im Vergleichsbeispiel 1 beschrieben hergestellt. Die Katalysatorträger wurden jedoch vorher mit Aluminiumchlorid imprägniert. Als Katalysatorträger wurde wieder der Alumosilikatträger KA 160 von Südchemie eingesetzt.

Hierzu wurde eine wäßrige Lösung aus Aluminiumchloridhydrat angesetzt, deren Konzentration so bemessen war, daß 0,11 Mol Aluminiumchloridhydrat pro 200 g Trägermaterial vorlagen. Nach diesem Imprägnierschritt wurden die Katalysatorträger bei 150 - 180°C für die Dauer von 2 Stunden getrocknet und calciniert.

Vor der Imprägnierung mit Kaliumacetat betrug die spezifische Oberfläche des Katalysators noch 140 - 150 m²/g. Durch die Aktivierung mit Kaliumacetat ging die spezifische Oberfläche des Katalysators auf 94 m²/g zurück.

Die CO-Adsorption des Katalysators vor der Aktivierung betrug 0,186 ml CO/g Katalysator. Die Bruchfestigkeit des aktivierten Katalysators wurde zu 78 N bestimmt. Seine edelmetallhaltige äußere Schale besaß eine Dicke von 0,3 mm.

### Beispiel 2:

Es wurde ein Palladium-Gold-Kaliumacetat-Katalysator wie im Vergleichsbeispiel 1 beschrieben hergestellt. Der Katalysatorträger wurde jedoch vorher erfindungsgemäß mit Zirkoniumacetat imprägniert. Als Katalysatorträger wurde der Alumosilikatträger KA 160 von Südchemie verwendet.

Es wurde eine wäßrige Lösung aus Zirkoniumacetat angesetzt, deren Konzentration so bemessen war, daß 0,11 mol Zirkoniumacetat pro 200 g Trägermaterial vorlagen. Nach diesem Imprägnierschritt wurde der Katalysatorträger bei 150 - 180°C für die Dauer von 2 Stunden getrocknet und calciniert. Ansonsten wurde der Katalysator wie im Vergleichsbeispiel 1 beschrieben hergestellt.

Vor der Imprägnierung mit Kaliumacetat betrug die spezifische Oberfläche des Katalysators noch 140 m²/g. Durch die Imprägnierung mit Kaliumacetat verringerte sich die spezifische Oberfläche des Katalysators auf 94 m²/g.

Die CO-Adsorption des Katalysators vor der Aktivierung mit Kaliumacetat lag bei 0,168 ml CO/g Katalysator. Die Bruchfestigkeit des aktivierten Katalysators betrug 70 N. Seine edelmetallhaltige äußere Schale besaß eine Dicke von 0,3 mm.

### Vergleichsbeispiel 2:

Es wurde ein konventioneller Palladium-Gold-Kaliumacetat-Katalysator analog zu Vergleichsbeispiel 1 auf einem Katalysatorträger aus pyrogener Kieselsäure (AEROSIL-Träger 350 von Degussa; spezifische Oberfläche 180 m²/g; Schüttdichte 490 g/l; gesamtes Porenvolumen 0,8 cm³/g; Tabletten von 6 mm Durchmesser und 5,5 mm Höhe) hergestellt. Die Konzentration der Imprägnierlösungen wurden so gewählt, daß der fertige Katalysator 2,71 g Palladium, 1,23 g Gold und 24,6 g Kaliumacetat pro Liter Schüttvolumen des Katalysatorträgers enthielt. Dies entsprach einer Konzentration von 0,55 Gew.-% Pd, 0,25 Gew.-% Au und 5,0 Gew.-% Kaliumacetat bezogen auf das Gewicht des eingesetzten Trägermaterials. Ansonsten wurde der Katalysator wie im Vergleichsbeispiel 1 beschrieben hergestellt.

Die CO-Adsorption des Katalysators vor der Aktivierung betrug 0,144 ml CO/g Katalysator. Seine Schalendicke wurde zu 0,5 mm bestimmt.

### Referenz - Beispiel 3:

Es wurde ein Palladium-Gold-Kaliumacetat-Katalysator wie im Vergleichsbeispiel 2 beschrieben hergestellt. Der Katalysatorträger wurde jedoch vorher erfindungsgemäß mit Aluminiumchloridhydrat imprägniert. Die Imprägnierung mit Aluminiumchloridhydrat wurde wie in Referenz - Beispiel 1 beschrieben vorgenommen. Als Katalysatorträger wurde der aus pyrogener Kieselsäure hergestellte Träger von Vergleichsbeispiel 2 verwendet.

Die CO-Adsorption des Katalysators vor der Aktivierung mit Kaliumacetat lag bei 0,314 ml CO/g Katalysator.

### Anwendungsbeispiel 1:

Aktivität und Selektivität der Katalysatoren aus den vorangegangenen Beispielen wurden während einer Langzeitprüfung über die Dauer von bis zu 200 Stunden gemessen. Die Aktivität der Katalysatoren steigt während einer Formierphase, die mehrere Stunden oder Tage betragen kann, kontinuierlich an und erreicht erst nach Abschluß dieser Phase einen konstanten Wert. Die in Tabelle 1 angegebenen Meßwerte wurden erst nach Abschluß der jeweiligen Formierphase gewonnen.

Zur Prüfung wurden die Katalysatoren der Vergleichsbeispiele 1 und 2, der Referenz - Beispiele 1 und 3 sowie des Beispiels 2 in einem mit Öl beheizten Strömungsrohrreaktor (Reaktorlänge 800 mm, Innendurchmesser 24,8 mm) bei Normaldruck und einer Raumgeschwindigkeit (GHSV = gas hourly space velocity) von 400 h⁻¹ mit der folgenden Gaszusammensetzung untersucht: 76,0 Vol.-% Ethen, 18,0 Vol.-% Essigsäure, 6,0 Vol.-% Sauerstoff.

Je nach Aktivität und Selektivität der Katalysatoren wurde die Reaktortemperatur im Temperaturbereich von 130 - 145 °C so eingeregelt, daß die Temperatur in der Mitte des Katalysatorbettes zwischen 150 und 160 °C lag.

Die Reaktionsprodukte wurden im Ausgang des Reaktors kondensiert und mittels Gaschromatographie auf ihre Zusammensetzung untersucht. Als Maß für die Katalysatoraktivität wurde die Raum-Zeit-Ausbeute des Katalysators in Gramm Vinylacetatmonomer pro Stunde und Liter Katalysatorvolumen (g VAM/(h · lₖₐₜ.)) bzw. in Gramm Vinylacetatmonomer pro Stunde und Kilogramm Katalysator (g VAM/(h · kg_{kat.})) bestimmt. Kohlendioxid, welches insbesondere durch die Verbrennung von Ethen gebildet wird, wurde im Abgas des Reaktors gemessen und zur Beurteilung der Katalysatorselektivität herangezogen.

In Tabelle 1 sind die Testergebnisse der auf den vorbehandelten Katalysatorträgern hergestellten Katalysatoren RB1, B2 und RB3 im Vergleich zu den auf den nicht vorbehandelten Katalysatorträgern hergestellten Katalysatoren (Vergleichskatalysatoren) VB1 und VB2 dargestellt.

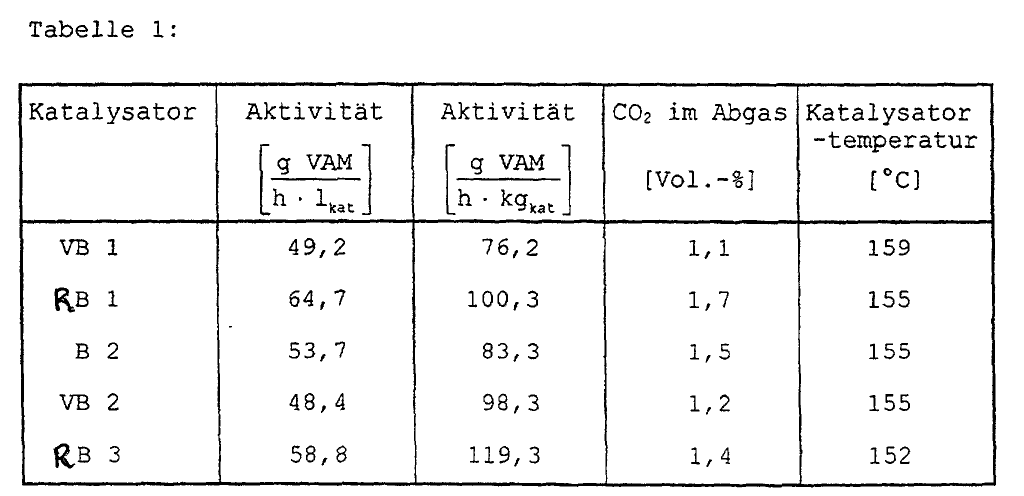

Die Beispiele zeigen, daß die erfindungsgemäßen Katalysatoren eine deutlich gesteigerte Raum-Zeit-Ausbeute an Vinylacetatmonomer bei vergleichbarer Bildung von CO₂ ermöglichen.

Der Katalysator auf dem Träger aus modifizierter pyrogener Kieselsäure Referenz - (Beispiel 3) weist gewichtsbezogen eine deutlich höhere Raum-Zeit-Ausbeute auf als die Katalysatoren auf dem Träger aus modifiziertem Alumosilikat KA 160 (Referenz - Beispiel 1 und Beispiel 2). Der Katalysator nach Referenz - Beispiel 3 ist darüberhinaus selektiver als der entsprechende Katalysator nach Referenz - Beispiel 1.

### Vergleichsbeispiel 3:

Es wurde ein konventioneller Palladium-Gold-Kaliumacetat-Katalysator auf dem Alumosilikatträger KA 160 hergestellt. Die Konzentration der Imprägierlösungen wurde so gewählt, daß der fertige Katalysator 3,3 g Palladium, 1,5 g Gold und 30 g Kaliumacetat pro Liter Schüttvolumen des Katalysatorträgers enthielt. Dies entsprach einer Konzentration von 0,55 Gew.-% Palladium, 0,25 Gew.-% Gold und 5,0 Gew.-% Kaliumacetat bezogen auf das Gewicht des eingesetzten Trägers.

In einem ersten Schritt wurde der Träger zunächst mit einer basischen Lösung aus Natriumhydroxid in Wasser imprägniert. Das Volumen der wäßrigen NaOH-Lösung entsprach 50 Prozent der Wasseraufnahme des trockenen Trägers. Nach der Imprägnierung mit Natriumhydroxid wurde der Träger unmittelbar ohne Zwischentrocknung mit einer wäßrigen Edelmetallösung aus Natriumpalladiumchlorid und Tetrachlorogoldsäure imprägniert, deren Volumen ebenfalls 50 % der Wasseraufnahmekapazität des trockenen Trägermaterials entsprach.

Nach einer Wartezeit von 1,5 Stunden zwecks Hydrolyse der Edelmetallverbindungen wurden die Trägerteilchen chlorfrei gewaschen.

Der Katalysator wurde getrocknet und wie in der EP 0 634 209 beschrieben bei 150°C in der Gasphase mit Formiergas reduziert. Danach wurde der Katalysator mit einer wäßrigen Kaliumacetat-Lösung imprägniert und erneut getrocknet. Die Trocknung wurde in der Gasphase mit Stickstoff durchgeführt.

Die Konzentration der basischen Lösung an Natriumhydroxid war so bemessen, daß sich auf den Trägerteilchen eine edelmetallhaltige Schale von 0,3 mm Dicke ausbildete.

### Vergleichsbeispiel 4:

Es wurde ein Palladium-Gold-Kaliumacetat-Katalysator wie im Vergleichsbeispiel 3 beschrieben hergestellt. Als Katalysatorträger wurde der Alumosilikatträger KA 160 verwendet. Der Katalysator wurde im Unterschied zu Vergleichsbeispiel 2 jedoch nicht mit Formiergas in der Gasphase, sondern in wäßriger Phase mit Hydrazin reduziert.

### Vergleichsbeispiel 5:

Es wurde ein Palladium-Gold-Kaliumacetat-Katalysator wie im Vergleichsbeispiel 3 beschrieben hergestellt. Als Katalysatorträger wurde der Alumosilikatträger KA 160 verwendet. Der Katalysator wurde im Unterschied zu Vergleichsbeispiel 3 jedoch nicht mit Formiergas, sondern mit Ethen in der Gasphase bei 150 °C reduziert.

### Referenz - Beispiel 4:

Es wurde ein Palladium-Gold-Kaliumacetat-Katalysator wie im Vergleichsbeispiel 3 hergestellt. Als Katalysatorträger wurde der Alumosilikatträger KA 160 verwendet. Der Katalysator wurde im Unterschied zu Vergleichsbeispiel 3 jedoch nicht bei 150 °C, sondern bei 450 °C in der Gasphase mit Formiergas reduziert.

### Anwendungsbeispiel 2

Aktivität und Selektivität der Katalysatoren aus den Vergleichsbeispielen 3, 4 und 5 und aus Referenz - Beispiel 4 wurden während einer Prüfung über die Dauer von bis zu 24 Stunden gemessen.

Die Katalysatoren wurden in einem mit Öl beheizten Strömungsrohrreaktor (Reaktorlänge 710 mm, Innendurchmesser 23,7 mm) bei Normaldruck und einer Raumgeschwindigkeit (GHSV) von 400 h⁻¹ mit der folgenden Gaszusammensetzung geprüft: 75 Vol.-% Ethen, 16,6 Vol.-% Essigsäure, 8,3 Vol.-% Sauerstoff. Die Katalysatoren wurden im Temperaturbereich von 120 bis 165 °C, gemessen im Katalysatorbett, untersucht.

Die Reaktionsprodukte wurden im Ausgang des Reaktors mittels on-line Gaschromatographie analysiert. Als Maß für die Katalysatoraktivität wurde die Raum-Zeit-Ausbeute des Katalysators in Gramm Vinylacetat-Monomer pro Stunde und Liter Katalysatorvolumen (g VAM/ (h·l_{Kat}.)) bzw. in Gramm Vinylacetat-Monomer pro Stunde und Kilogramm Katalysator (g VAM/(h'kg_{Kat.})) bestimmt. Kohlendioxid, das insbesondere durch die Verbrennung von Ethen gebildet wird, wurde ebenfalls bestimmt und zur Beurteilung der Katalysatorselektivität herangezogen.

In Tabelle 2 sind die Untersuchungsergebnisse an den Katalysatoren aus den Vergleichsbeispielen 3, 4 und 5 sowie aus Referenz - Beispiel 4 dargestellt.

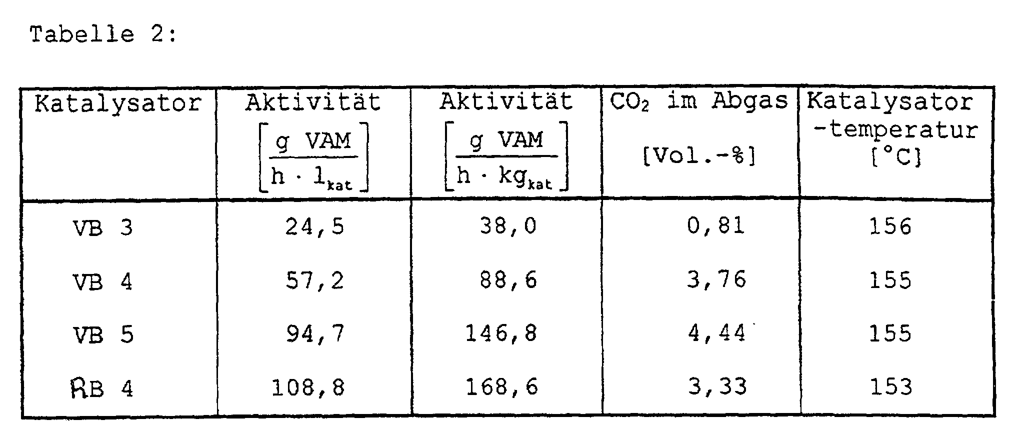

Das Referenz - Beispiel 4 zeigt, daß der bei 450°C mit Formiergas reduzierte Katalysator eine deutlich gesteigerte Raum-Zeit-Ausbeute an Vinylacetatmonomer ermöglicht.

Wird der Katalysator wie im Vergleichsbeispiel 3 beschrieben bei 150 °C mit Formiergas in der Gasphase reduziert, so ergibt sich lediglich eine sehr geringe Raum-Zeit-Ausbeute an Vinylacetatmonomer. Die Reduktion in flüssiger Phase mit Hydrazin (Vergleichsbeispiel 4) bzw. mit Ethen in der Gasphase bei 150 °C (Vergleichsbeispiel 5) führt zu Katalysatoren mit deutlich verbesserter Raum-Zeit-Ausbeute an Vinylacetatmonomer. Wird der Katalysator nun wie im Referenz - Beispiel 4 beschrieben bei 450 °C in der Gasphase mit Formiergas reduziert, so kann die Raum-Zeit-Ausbeute an Vinylacetatmonomer überraschenderweise nochmals deutlich gesteigert werden. Im Vergleich zu den weniger selektiven Katalysatoren aus den Vergleichsbeispielen 4 und 5 konnte auch die Selektivität deutlich gesteigert werden.

### Vergleichsbeispiel 6:

Es wurde ein Palladium-Gold-Kaliumacetat-Katalysator auf dem Alumosilikatträger KA 160 wie im Referenz - Beispiel 4 beschrieben hergestellt. Der Katalysatorträger wurde jedoch vorher mit Lanthannitrat imprägniert.

Hierzu wurde eine wäßrige Lösung aus Lanthannitrat angesetzt, deren Konzentration so bemessen war, daß 0,11 mol Lanthannitrat pro 200 g Trägermaterial vorlagen. Nach diesem Imprägnierschritt wurde der Katalysatorträger bei 150 - 180 °C für die Dauer von 2 Stunden getrocknet und calciniert.

### Vergleichsbeispiel 7:

Es wurde ein Palladium-Gold-Kaliumacetat-Katalysator auf dem Alumosilikatträger KA 160 wie im Vergleichsbeispiel 6 beschrieben hergestellt. Anstelle mit Lanthannitrat wurde der Katalysatorträger mit Wismutchlorid vorbehandelt.

### Beispiel 5:

Es wurde ein Palladium-Gold-Kaliumacetat-Katalysator auf dem Alumosilikatträger KA 160 wie im Vergleichsbeispiel 6 beschrieben hergestellt. Anstelle mit Lanthannitrat wurde der Katalysatorträger mit Titan(III)-chlorid vorbehandelt.

### Beispiel 6:

Es wurde ein Palladium-Gold-Kaliumacetat-Katalysator auf dem Alumosilikatträger KA 160 wie im Vergleichsbeispiel 6 beschrieben hergestellt. Anstelle mit Lanthannitrat wurde der Katalysatorträger mit Zirkoniumacetat vorbehandelt.

Die Katalysatoren der Beispiele RB4, B5, B6 und der Vergleichsbeispiele VB6 und VB7 wurden wie im Anwendungsbeispiel 2 beschrieben auf Aktivität und Selektivität geprüft. Die Untersuchungsergebnisse sind in Tabelle 3 zusammengestellt.

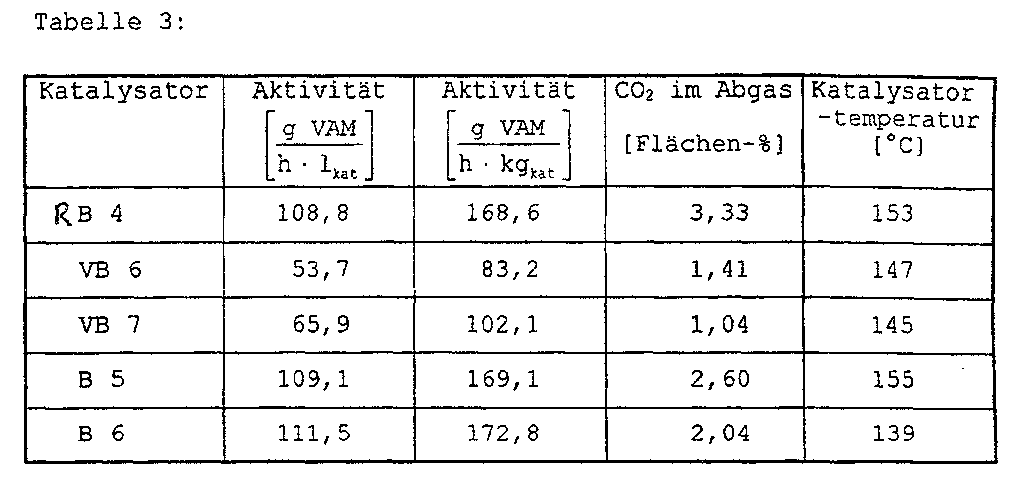

In Tabelle 3 sind die Katalysatortemperaturen angegeben, bei denen die Katalysatoren die jeweils höchste Raum-Zeit-Ausbeute ergaben.

Die Katalysatoren aus Vergleichsbeispiel 6 (Lanthan modifizierter Katalysatorträger) und aus Vergleichsbeispiel 7 (Wismut modifizierter Katalysatorträger) zeigen wesentlich schlechtere Leistungsdaten als die Katalysatoren der Beispiele RB4, B5 und B6. Die Katalysatoren aus Beispiel 5 (Titan modifizierter Katalysatorträger) und aus Beispiel 6 (Zirkon modifizierter Katalysatorträger) sind noch etwas aktiver und selektiver als die Katalysatoren aus Referenz - Beispiel 4 auf dem nicht modifizierten Katalysatorträger. Die Katalysatoren von Beispiel 6 (Zirkon modifizierter Katalysatorträger) ergeben die höchste Raum-Zeit-Ausbeute bei einer vergleichsweise sehr niedrigen Temperatur von 139 °C.

### Referenz - Beispiel 7:

Es wurde ein Palladium-Gold-Kaliumacetat-Katalysator auf dem Alumosilikatträger KA 160 nach Referenz- Beispiel 4 hergestellt.

Im Unterschied zum Katalysator nach Referenz - Beispiel 4 wurde der Katalysator im vorliegenden Fall jedoch mit einem Edelmetallgehalt von 1,2 Gew.-% Palladium, 0,5 Gew.-% Gold und 5 Gew.-% Kaliumacetat hergestellt.

### Beispiel 8:

Es wurde ein Palladium-Gold-Kaliumacetat-Katalysator auf dem Alumosilikatträger KA 160 nach Referenz - Beispiel 4 hergestellt.

Im Unterschied zum Katalysator nach Referenz - Beispiel 4 wurde der Katalysator im vorliegenden Fall jedoch mit einem Edelmetallgehalt von 1,2 Gew.-% Palladium, 0,5 Gew.-% Gold und 5 Gew.-% Kaliumacetat hergestellt. Zusätzlich wurde der Katalysatorträger vor der Belegung mit Edelmetall mit Zirkoniumacetat wie in Beispiel 2 beschrieben imprägniert.

### Referenz- Beispiel 9:

Es wurde ein Palladium-Gold-Kaliumacetat-Katalysator auf dem Alumosilikatträger KA 160 nach Beispiel 8 hergestellt.

Abweichend von Beispiel 8 wurde der Katalysatorträger mit Aluminiumchloridhydrat wie in Beispiel 1 beschrieben vorbehandelt.

In Tabelle 4 sind die Testergebnisse der auf den vorbehandelten beziehungsweise modifizierten Katalysatorträgern hergestellten Katalysatoren im Vergleich zu dem auf dem nicht vorbehandelten Katalysatorträger hergestellten Katalysator aus Referenz - Beispiel 7 dargestellt. Die Katalysatoren wurden wie im Anwendungsbeispiel 2 beschrieben untersucht.

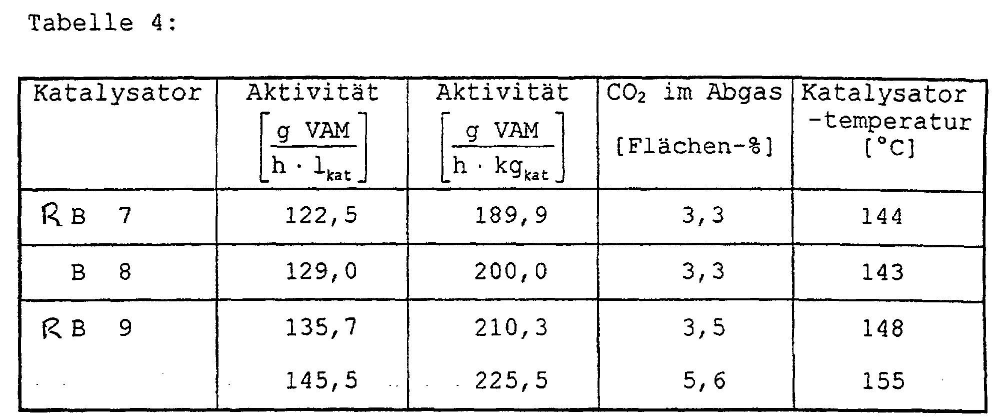

Auch das Beispiel 8 und des Referenz - Beispiel 9 zeigen, daß Katalysatoren auf modifizierten Katalysatorträgern bei vergleichbarer Selektivität eine deutlich erhöhte Raum-Zeit-Ausbeute aufweisen.

### Referenz - Beispiel 10:

Es wurde eine Palladium-Gold-Kaliumacetat Katalysator nach Referenz - Beispiel 4 hergestellt. Als Katalysatorträger wurde ein Träger aus pyrogener Kieselsäure wie in Vergleichsbeispiel 2 eingesetzt. Im Unterschied zum Katalysator nach Beispiel 4 wurde der Katalysator in diesem Fall mit einem Edelmetallgehalt von 1,2 Gew.-% Palladium, 0,5 Gew.-% Gold und 5 Gew.-% Kaliumacetat hergestellt.

### Beispiel 11:

Es wurde ein Palladium-Gold-Kaliumacetat-Katalysator nach Referenz - Beispiel 10 hergestellt.

Im Unterschied zum Beispiel 10 wurde der Katalysatorträger vor der Belegung mit Edelmetall mit Zirkoniumacetat wie in Beispiel 2 beschrieben imprägniert.

### Beispiel 12:

Es wurde ein Palladium-Gold-Kaliumacetat-Katalysator nach Beispiel 11 hergestellt.

Im Unterschied zum Katalysator nach Beispiel 11 wurde der Katalysatorträger nach der Vorbehandlung mit Zirkoniumacetat nicht bei 150 bis 180° C getrocknet und calciniert, sondern zunächst bei 120°C getrocknet und dann bei 400°C für die Dauer von 1 bis 2 Stunden calciniert.

In Tabelle 5 sind die Testergebnisse der auf den vorbehandelten Katalysatorträgern hergestellten Katalysatoren im Vergleich zu dem auf dem nicht vorbehandelten Katalysatorträger hergestellten Katalysator aus Referenz - Beispiel 10 dargestellt. Die Katalysatoren wurden wie im Anwendungsbeispiel 2 beschrieben untersucht.

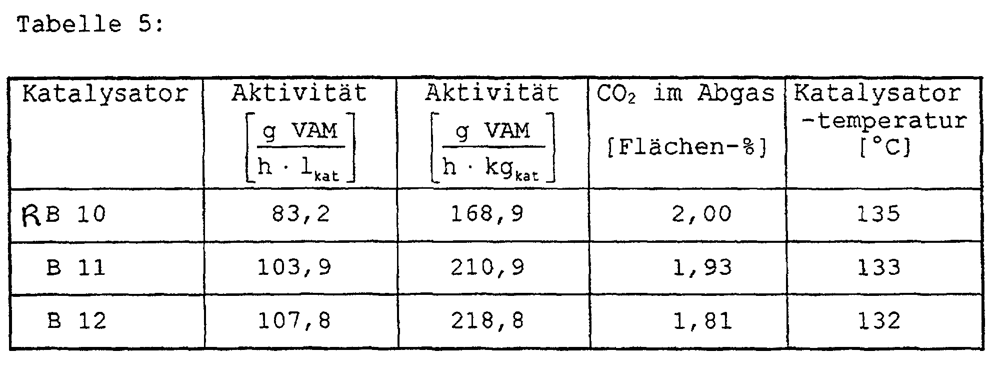

Die Katalysatortestergebnisse in Tabelle 5 zeigen, daß die Katalysatoren nach Beispiel 11 und 12 auf modifizierten Katalysatorträgern im Vergleich zu dem Katalysator aus Referenz - Beispiel 10 auf einem nicht modifizierten Katalysatorträger sowohl eine deutlich höhere Aktivität als auch eine niedrigere CO₂-Bildung und damit eine höhere Selektivität aufweisen.

## Patentansprüche

1. Trägerkatalysator für die Produktion von Vinylacetatmonomer enthaltend auf einem Träger aus Siliziumdioxid, Alumosilikat oder Aluminiumoxid als katalytisch aktive Komponenten Palladium, Gold und Alkaliacetat,
**dadurch gekennzeichnet,**
**daß** der Träger zusätzlich mindestens ein Element aus der Gruppe Zirkon und/oder Titan enthält.

2. Trägerkatalysator nach Anspruch 1,
**dadurch gekennzeichnet,**
**daß** die zusätzlichen Elemente in einer Konzentration von 0,1 bis 25 Gew.-% bezogen auf das Gewicht des Trägers vorliegen.

3. Verfahren zur Herstellung eines Trägerkatalysators für die Produktion von Vinylacetatmonomer durch Imprägnieren des Trägers mit einer basischen Lösung und einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln,
**dadurch gekennzeichnet,**
**daß** der Träger in einer Vorbehandlung mit Salzen, welche als Kationen Elemente der Gruppe Zirkon und/oder Titan und als Anionen Elemente der Gruppe VIIA oder komplexe Anionen wie Nitrat, Sulfat, Carbonat oder Anionen organischer Säuren wie Acetat und Lactat enthalten, oder mit Organometallverbindungen, insbesondere Alkoholaten, dieser Elemente imprägniert, bei erhöhten Temperaturen getrocknet und anschließend bei Temperaturen von 160 bis 800°C calciniert werden.

4. Verfahren nach Anspruch 3,
**dadurch gekennzeichnet,**
**daß** der Träger durch die Vorbehandlung mit den Kationen in einer Menge von 0,1 bis 25 Gew.-% bezogen auf das Gewicht des Trägers vorbelegt wird.

5. Verfahren nach Anspruch 3 oder 4,
**dadurch gekennzeichnet,**
**daß** die Reduktion im Formiergas bei Temperaturen zwischen 300 und 550, bevorzugt zwischen 350 und 500°C durchgeführt wird.

6. Verwendung der Katalysatoren nach einem der vorstehenden Ansprüche für die Produktion von Vinylacetatmonomer durch Umsetzung von Ethylen, Essigsäure und Sauerstoff an dem Katalysator in der Gasphase bei erhöhtem Druck.

## Claims

1. Support catalyst for the production of vinyl acetate monomer, containing on a support of silicon dioxide, alumosilicate or aluminium oxide palladium, gold and alkali acetate as the catalytically active components,
**characterised in that**
the support additionally contains at least one element from the group zirconium and/or titanium.

2. Support catalyst according to claim 1,
**characterised in that**
the additional elements are present in a concentration of from 0.1 to 25 wt.%, based on the weight of the support.

3. Process for the preparation of a support catalyst for the production of vinyl acetate monomer, by impregnating the support with a basic solution and with a solution containing gold salts and palladium salts, the impregnation being carried out simultaneously or in succession, with or without intermediate drying, washing the support in order to remove any chloride components that may be present and, before or after the washing step, reducing the insoluble compounds that have precipitated on the support, drying the catalyst precursor so obtained, and impregnating it with alkali acetates or with alkali compounds that are converted wholly or partially into alkali acetates under the reaction conditions in the production of vinyl acetate monomer,
**characterised in that**
the support is impregnated in a pretreatment step with salts that contain as cations elements of the group zirconium and/or titanium and as anions elements of group VIIA or complex anions such as nitrate, sulfate, carbonate or anions of organic acids such as acetate and lactate, or with organometallic compounds, especially alcoholates, of such elements, is dried at elevated temperatures and is then calcined at temperatures of from 160 to 800°C.

4. Process according to claim 3,
**characterised in that**
the support, as a result of the pretreatment, is pre-coated with the cations in an amount of from 0.1 to 25 wt.%, based on the weight of the support.

5. Process according to claim 3 or 4,
**characterised in that**
the reduction is carried out in forming gas at temperatures of from 300 to 550°C, preferably from 350 to 500°C.

6. Use of catalysts according to any one of the preceding claims in the production of vinyl acetate monomer by reaction of ethylene, acetic acid and oxygen on the catalyst in the gas phase at elevated pressure.

## Revendications

1. Catalyseur sur support pour la production de monomère d'acétate de vinyle contenant comme composants actifs catalytiquement du palladium, de l'or et un acétate de métal alcalin sur un support en dioxyde de silicium, en aluminosilicate où en un oxyde d'aluminium,
**caractérisé en ce que**
le support renferme en supplément au moins un élément choisi dans le groupe du zirconium et/ou du titane.

2. Catalyseur sur support selon la revendication 1,
**caractérisé en ce que**
les éléments supplémentaires sont présents à une concentration allant de 0,1 à 25 % en poids rapporté au poids du support.

3. Procédé de préparation d'un catalyseur sur support pour la production de monomère d'acétate de vinyle, par imprégnation du support avec une solution basique et avec une solution contenant un sel d'or et un sel de palladium, dans lequel l'imprégnation s'effectue simultanément ou l'une après l'autre avec ou sans étape intermédiaire de séchage, lavage du support pour l'élimination de fractions de chlorures éventuellement présents, et par réduction des composés insolubles précipités sur le support avant ou après le lavage, le séchage du stade préliminaire de catalyseur ainsi obtenu, et par imprégnation avec des acétates de métal alcalin ou des composés alcalins qui se transforment dans les conditions de réaction au cours de la production de monomère d'acétate de vinyle totalement ou partiellement en acétates de métal alcalin,
**caractérisé en ce qu'**
on imprègne le support dans un prétraitement avec des sels qui contiennent comme cations des éléments choisis dans le groupe du zirconium et/ou du titane et comme anions des éléments du groupe VIIa ou des anions complexes comme un nitrate, un sulfate, un carbonate, ou des anions d'acide organique comme un acétate et un lactate, ou bien avec des composés organométalliques, en particulier avec des alcoolates, de ces éléments, on sèche à des températures accrues et ensuite on calcine à des températures allant de 160 à 800°C.

4. Procédé selon la revendication 3,
**caractérisé en ce qu'**
on prérevêt le support par le prétraitement avec les cations en une quantité allant de 0,1 à 25 % en poids, rapporté au poids du support.

5. Procédé selon la revendication 3 ou la revendication 4,
**caractérisé en ce qu'**
on effectue la réduction dans un gaz de formage à des températures comprises entre 300 et 550°C, de préférence entre 350 et 500°C.

6. Utilisation des catalyseurs selon l'une quelconque des revendications précédentes pour la production de monomère d'acétate de vinyle par réaction d'éthylène, d'acide acétique et d'oxygène sur le catalyseur en phase gazeuse à pression accrue.
